# EUROPEAN PATENT SPECIFICATION

(11) **EP 0 615 433 B1**
(45) Date of publication and mention of the grant of the patent: **12.03.1997**
(21) Application number: 92920164.8
(22) Date of filing: 08.09.1992
(51) Int. Cl.: A61F 13/58, C09J 153/02

(54) **A THERMOPLASTIC ADHESIVE COMPOSITION AND CORRESPONDING ARTICLE**
THERMOPLASTISCHE KLEBESTOFF-ZUSAMMENSETZUNG UND ENTSPRECHENDER ARTIKEL
COMPOSITION ADHESIVE THERMOPLASTIQUE ET ARTICLE CORRESPONDANT

(30) Priority: 02.12.1991 US 801699
(43) Date of publication of application: 21.09.1994
(73) Proprietor: H.B. Fuller Licensing & Financing, Inc., St. Paul, Minnesota 55110-5132 (US)
(72) Inventor: REENTS, Lisa, L., Dayton, Minnesota 55327 (US); BUNNELLE, William, L., Ham Lake, Minnesota 55304 (US)
(74) Representative: Maiwald, Walter, Dr. Dipl.-Chem.
(86) International application number: US9207580
(87) International publication number: WO9310734

(56) References cited:
- US-A- 3 993 613
- US-A- 4 345 349
- US-A- 5 028 646

## Description

### Field of the Invention

The invention relates to a hot melt adhesive and to a disposable article utilizing the adhesive. The invention more particularly relates to a disposable article such as a sanitary napkin, an incontinent pad, a bed pad, or a feminine pad. The adhesive of the invention is utilized as a positioning adhesive through which the napkin or pad is adhered to undergarments made of cotton, silk or man-made fibers such as nylon.

### Background of the Invention

The typical commercial disposable article adhesives use a plasticizing oil and a tackifying resin in combination with typically a styrene-ethylene/butylene-styrene (S-EB-S) or styrene-isoprene-styrene (S-I-S) linear (A-B-A) block or a multi-block (A-B-A-B-A-B) copolymer having a molecular weight less than about 140,000. Such conventional commercial adhesives are typically formulated to generally contain greater than 20 wt-% S-I-S or S-EB-S, A-B-A block copolymer or a multi-block (A-B-A-B-A-B) copolymer, generally 25 to 35 wt-% with 40 to 60 wt-% tackifier and 25 to 35 wt-% oil. To date, amounts of block copolymer ranging from 20-35 wt-% of the adhesive have been required to maintain adequate cohesive strength and adhesion properties in linear A-B-A block copolymer systems. The use of this amount of A-B-A rubber in these formulas result in high costs when compared to other adhesive systems. Additionally, such an adhesive with a high polymer content can result in a product viscosity that is in excess of the maximum viscosity useful for application temperatures lower than 176,6°C (350°F). It is desirable to apply positioning adhesives at temperatures lower than 176,6°C (350°F) for lower basis weight or heat sensitive substrates. To date, adhesives with a lower polymer level (i.e. about 20 wt-%) and a viscosity in the applicable range have lost cohesive strength which results in adhesive transfer to the undergarment material, and produce unacceptably high peel strengths in the attachment mode.

A different type of adhesive is disclosed in Doss et al, U.S. Pat. No. 3,993,613, which teaches an adhesive composition comprising about 10 parts by weight of a rubbery radial teleblock copolymer and a blended tackifier made by mixing a polyterpene, a polyvinyl aromatic or other tackifying resins with a polyalkylene tackifying copolymer. Additionally, Taylor et al, U.S. Pat. No. 4,212,910 teaches a PET bottle base adhesive having 20-40 percent by weight of a low molecular weight radial block polymer, 30-59% tackifier and 20-40% wax or oil.

Collins et al, U.S. Pat. No. 4,136,699 teaches an S-EB-S linear A-B-A block copolymer based construction and positioning adhesive for absorbent articles. Schmidt, Jr. et al., U.S. Pat. No. 4,526,577 teaches the use of S-B-S linear A-B-A block and linear A-B-A-B-A-B multi-block copolymers in hot melt adhesives in the manufacture of disposable laminates using multi-line extrusion adhesive application technology. Raykovitz et al., U.S. Pat. No. 4,704,110 teaches an absorbent article utilizing an adhesive with an S-B-S multi-block A-B-A-B-A-B copolymer. Raykovitz et al., generally discloses radial or teleblock block copolymers but fails to disclose any particulars of such polymers including molecular weight, percent midblock, percent di-block, molecular weight distribution, or other polymer indicia necessary for adhesive properties and formulation. Further, Raykovitz et al. teaches that the adhesive material should contain 20 to 40 percent by weight of the linear S-B-S block or linear multi-block copolymer. Additionally, the Raykovitz et al. patent prefers relatively high concentrations of styrene in the range of 38 to 55 parts styrene per 100 parts of copolymer. The typical S-EB-S and S-B-S block copolymers identified by the Collins et al. and the Raykovitz et al. patents include linear A-B-A block or A-B-A-B-A-B multiblock copolymers which range in molecular weight from about 100,000 to 150,000 as measured by Gel Permeation Chromatography with the values reported relative to polystyrene standards. These adhesives contain either Kraton G-1650, a linear S-EB-S block copolymer available from Shell Chemical, or Stereon 840A, a linear multi-block S-B-S copolymer available from Firestone. Both of these polymers have molecular weights in the range of 100,000.

In conventional production of sanitary napkins, such hot melt adhesives as mentioned above are typically applied at a temperature of about 176,6°C (350°F) onto a release liner and subsequently transferred onto a workpiece, typically a polyethylene or polypropylene film. The viscosity curves of these typical adhesives do not allow for direct application to many of the substrates used in the manufacturing of these sanitary devices, in particular, to lower basis weight or heat sensitive substrates. This is due to the fact that the viscosities are too high at temperatures of less than 176,6°C (350°F) to allow for a smooth, even application of the adhesive and at temperatures above 176,6°C (350°F), many of the substrates can distort, melt or otherwise be damaged by the heat of the adhesive.

A positioning adhesive is directed toward personal hygiene items such as sanitary napkins. The positioning adhesive is located on an outside surface of the hygiene item. A release liner is adhered to the adhesive on the hygiene item, and the item is packaged for consumer use. Upon use, the liner is removed from the personal hygiene item and the item is positioned on the user's undergarment. Often times, the user initially positions the hygiene item incorrectly, decides to change undergarments or is just removing a soiled hygiene item. If the adhesive is too strong, removal of the hygiene device can be difficult. Further, unwanted adhesive residue will be left on the garment. If the adhesive is too weak, either the hygiene item will slip in place, or it will not readhere. Thus, the adhesive must have the correct properties to be an effective, undergarment adhesive. Furthermore, the personal hygiene item must be easy to manufacture. This requires a broad temperature range through which the adhesive has flow properties such that it can be processed and applied to personal hygiene items. However, the adhesive cannot soften when exposed to typical use temperatures.

Thus, the adhesive must have strong, but not overly strong initial peel strength. A subsequent strong peel strength which allows the adhesive to readhere to different fabrics after removal is also desirable. Further, the adhesive must have a wide temperature window for application of the adhesive to the hygiene item, and the adhesive should be able to be applied directly to thin gauge polymeric film without a need to transfer the adhesive.

### Summary of the Invention

The present adhesive exhibits superior positioning properties when compared to the prior art adhesives, including a viscosity profile which results in a wide range of application temperatures and methods. Viscosities which are low at temperatures less than 176,7°C (350°F) allow for direct application to low basis weight and heat sensitive substrates.

The adhesive comprises:
(a) about 6 to 25 percent by weight of a radial block copolymer having a molecular weight of at least about 160,000 as measured by Gel Permeation Chromatography relative to polystyrene standards, and having the formula:

   (A-B)ₙ-Y

   wherein Y is a multivalent coupling agent, A comprises a polyvinyl substituted aromatic block, B comprises a polybutadiene rubbery midblock and n is an integer of 3 to 10;
(b) about 30 to 70 percent by weight of a compatible tackifying resin selected from the group consisting of a C₅ resin, a styrenated C₅ resin, a styrenated terpene resin, a fully hydrogenated resin, a partially hydrogenated resin, a rosin derivative, a styrenated terpene resin and mixtures thereof; and
(c) about 10 to 40 percent by weight of a plasticizing oil.

### Detailed Description of the Invention

We have found that surprisingly low levels of a radial block copolymer having a molecular weight (as measured by Gel Permeation Chromatography and the values reported relative to polystyrene standards) of greater than about 140,000, preferably greater than 200,000, can be used to obtain a high level of initial bond strength, a low viscosity profile, and can resist adhesive transfer when subjected to typical use temperatures and pressures. The adhesive has a wide temperature window through which the viscosity of the adhesive is low enough such that it can be applied directly to a polymeric film at temperatures considerably lower than previously obtainable with prior art positioning adhesives.

The radial block copolymer can be blended in amounts that are surprisingly small in comparison to the prior art adhesives, as represented by Raykovitz et al, U.S. Pat. No. 4,704,110, with appropriate tackifying resins and plasticizing oils. The radial block copolymers of the invention can optionally be combined with linear A-B-A (both S-I-S and S-B-S) block copolymers to produce an adhesive having superior adhesion and cohesion. The total amount of copolymer is generally between 6 to 25 percent by weight. The preferred is about 8 to 22 percent by weight. The most preferred is about 10 to 19 percent by weight. The preferred composition does not employ an additional linear block copolymer. However, if a linear block copolymer is employed, the total copolymer component is not to exceed 19 percent on account of the viscosity profile as described above. The adhesive has a useful viscosity profile at application temperatures which typically range from 121,1-176,6°C (250-350° F).

Radial (or teleblock) copolymers having a molecular weight in excess of about 200,000 are known and are described in the Shell brochure for **KRATON 1184**. An inspection of the brochure indicates that the material is primarily used as a flexibilizing agent for bituminous compositions such as asphalt. Other copolymers of this type are available from Enichem Elastomers Americas, Inc. and are known under the trade name of **Europrene Sol T**, and also from **Fina** and are known under the trade name of **Finaprene**. The radial or teleblock copolymers having a molecular weight above 140,000 can present significant difficulties in adhesive preparation to manufacturing personnel. While a laboratory blending apparatus can be used to manufacture adhesives in small laboratory scale amounts from such radial block copolymers, typical single stage hot melt manufacturing or plant blending techniques, utilized in preparation of prior art adhesives made of typical A-B-A block copolymers and A-B-A-B-A-B multi-block copolymers as represented by Schmidt, Jr., U.S. Pat. No. 4,256,577, fail to adequately blend some of the high molecular weight radial block copolymers, such as **Kraton 1184**, with tackifiers or plasticizers into useful adhesive compositions.

Further, the high molecular weight of the radial block copolymers when used at a concentration found in typical adhesive formulations (about 25 percent by weight and more) results in a viscosity that exceeds production specifications or machine application specifications in typical extrusion and slot die coating equipment.

The radial block copolymers useful in manufacturing the adhesives of the present invention have a molecular weight in excess of about 160,000 and have the general formula:

(A-B)ₙₗ-Y

wherein A is a glassy block of a polymerized vinyl substituted aromatic monomer, B is a saturated or unsaturated rubbery block of a polymerized diene having 4-12 carbon atoms, Y is a polyfunctional coupling agent residue, and **n** is an integer of at least 3, preferably 3 to 10, most preferably 3 to 5. The end A or mid B blocks can be homo or copolymers of related monomers. Further, at the transition between the blocks some random copolymerization of the A monomers with B monomers can exist.

The preferred radial block copolymers have the general formulae

A - B - Y - (B - A)ₙ

wherein **n** is preferably 1-3, but can be more depending on the multifunctional coupling agent. The A block comprises styrene and the B block comprises butadiene, isoprene, or mixtures thereof which can be hydrogenated. The preferred radial block copolymers contain preferably 15 to 45 wt-%, most preferably 25 to 35 wt-% of styrene. The average molecular weight of the preferred rubbery block copolymers is preferably about 160,000. Most preferably the molecular weight of the block copolymers used in the adhesives of the instant invention is greater than about 200,000. There are different methods used for measuring molecular weight. The method used for determining molecular weights for this invention was Gel Permeation Chromatography and the weight average molecular weights are relative to polystyrene standards. A table may be found below indicating the difference in molecular weight between polymers used in conventional positioning adhesives and the polymers of the instant invention:

| **MOLECULAR WEIGHT COMPARISON** | | | |
|---|---|---|---|
| **CONVENTIONAL POLYMERS** | | **POLYMERS OF THE INVENTION** | |
| Kraton G 1652 | 75,000 | Fina 401 | 260,000 |
| Kraton G 1650 | 103,000 | Fina 411 | 385,000 |
| Stereon 840A | 132,000 | Sol T 161A | 540,000 |
| Stereon 845 | 148,000 | Sol T 161B | 370,000 |
| | | Sol T 161C | 316,000 |
| | | Sol T 172 | 260,000 |
| | | Kraton D 1184 | 400,000 |
| | | Kraton D 4158 | 388,000 |

A radial block copolymer useful for the present invention includes a block copolymer available under the trade designation **Europrene Sol T 161C**, commercially available from Enichem Elastomers Americas, Inc., and having a molecular weight in excess of 160,000. The block copolymer is a multi-arm radial SBS Polymer which is 30% styrene by weight. Other useful radial block copolymers include **Kraton D 4158**, **Europrene SOL T 172**, **SOL T 161A**, **SOL T 161B**, **Kraton D 1184**. It should be noted that both the **Europrene SOL T 172** and the **Kraton D 4158** contain about 30% by weight of an extending oil.

A-B-A block copolymers can be used with the radial block polymers. The A blocks of such A-B-A block copolymer comprise blocks of polymerized vinyl substituted aromatic monomers and the B blocks comprise polymerized diene having 4-12 carbon atoms. Preferably the A-B-A block copolymers are made of A blocks comprising polymerized styrene and B blocks comprising polymerized butadiene, isoprene or mixtures thereof. Such copolymers typically have a molecular weight in the range of about 70,000-140,000 and have from about 12 to 35 wt-% styrene. Such linear and multi-block copolymers are available from Shell Chemical Company, Enichem, Fina and Firestone.

We have found that the adhesive's excellent properties can be improved by immobilizing the oil component of the adhesive. We believe that migration of oil and other low molecular weight components from the adhesive mass to the bond line between the adhesive and an undergarment can cause an unacceptable residue to be left on the undergarment. Immobilizing the oil in the adhesive prevents its migration and therefore increases bond strength. This can be achieved using at least two mechanisms. Low molecular weight polyethylene or its derivatives can be used to gel the oil within the adhesive. The gel effectively locks the oil in place within the adhesive mass, preventing its migration. Additionally, highly oil compatible tackifying agents can be used which through compatibility tend to prevent migration.

The polyfunctional coupling agents (Y) are well known coupling agents for use in the manufacture of the radial block copolymers of the invention. Such coupling agents and the preparations of the rubbery copolymers useful in the adhesive compositions of the invention are described in detail in a variety of patents including U.S. Pat. No. 3,639,521 and others.

### Tackifying Resin

The adhesives of the invention contain a tackifying resin in combination with a thermoplastic block copolymer and a plasticizer. Tackifying resins useful in the adhesives of the invention comprise rosin derivatives including wood rosin, tall oil, tall oil derivatives, rosin ester resins, natural and synthetic terpenes, aliphatic or mixed aliphatic-aromatic tackifying resins and partially or fully hydrogenated resins.

Aromatic monomers useful in forming the aliphatic aromatic resin compositions of this invention can be prepared from any monomer containing substantial aromatic qualities and a polymerizable unsaturated group. Typical examples of such aromatic monomers include the styrenic monomers, styrene, alphamethyl styrene, vinyl toluene, methoxy styrene, tertiary butyl styrene, chlorostyrene, etc., indene monomers including indene, methyl indene and others. Aliphatic monomers are typical natural and synthetic terpenes which contain C₅ and C₆ cyclohexyl or cyclopentyl saturated groups that can additionally contain a variety of substantial aromatic ring substituents. Aliphatic tackifying resins can be made by polymerizing a feed stream containing sufficient aliphatic monomers such that the resulting resin exhibits aliphatic characteristics. Such feed streams can contain other aliphatic unsaturated monomers such as 1,3-butadiene, cis-1,3-pentadiene, trans-1,3-pentadiene, 2-methyl-1,3-butadiene, 2-methyl-2-butene, cyclopentadiene, dicyclopentadiene, terpene monomers as well as others. Mixed aliphatic aromatic resins contain sufficient aromatic monomers and sufficient aliphatic monomers and optionally other C₃-C₈ unsaturated monomers to produce a resin having both aliphatic and aromatic character. Resins from an aromatic feed source are also desirable. The article by Davis, The Chemistry of C₅ Resins, discusses synthetic C₅ resin technology.

The adhesive compositions of the invention can contain rosin and rosin derivatives as a tackifying agent. Rosin is a solid material that occurs naturally in the oleo rosin of pine trees and typically is derived from the oleo resinous exudate of the living tree, from aged stumps and from tall oil produced as a by-product of kraft paper manufacture. After it is obtained the rosin can be treated by hydrogenation, dehydrogenation, polymerization, esterification, and other post treatment processes. Rosin is typically classed as a gum rosin, a wood rosin, or as a tall oil rosin which indicate its source. The materials can be used unmodified, in the form of esters of polyhydric alcohols, and can be polymerized through the inherent unsaturation of the molecules. Materials are commercially available and can be blended into the adhesive compositions using standard blending techniques. Representative examples of such rosin derivatives include pentaerythritol esters of tall oil, gum rosin, wood rosin, or mixtures thereof.

Representative examples of such aliphatic resins include hydrogenated synthetic C₉ resins, synthetic branched and unbranched C₅ resins and mixtures thereof. Representative examples of such aromatic modified tackifying resins include styrenated terpene resins, styrenated C₅ resins or mixtures thereof. The selection of tackifying resins is often based on the nature of the B or midblock radial block copolymer. Rosin derivatives are best for S-I-S/S-B-S blends and can be used with either S-I-S or S-B-S alone. Hydrogenated C₉ or straight aliphatic resins are preferred for S-I-S copolymers. For S-B-S copolymers, fully hydrogenated resins, partially hydrogenated resins, styrenated terpenes, rosin esters or aliphatic and/or aromatic hydrocarbons are preferred.

Commercially available tackifying resins useful for the present invention include **Eastotac H** series resins available from Eastman Chemical, **Unitac RLM** available from Union Camp, **Sylvatac 1100** series available from Arizona Chemical Company, **Wingtack** resins from Goodyear, and **Zonatac Resins** from Arizona Chemical Company. The amount of tackifying resin is about 30 to 70 percent by weight and preferably about 40 to 65 percent by weight.

### Plasticizing Oils

Plasticizing oils are used in the positioning adhesives of the invention. Such oils are primarily hydrocarbon oils low in aromatic content. Preferably the oils are paraffinic or naphthenic in character. The oils are preferably low in volatility, are clear and have as little color and odor as possible. The use of a plasticizing oil of this invention also contemplates the use of olefin oligomers, low molecular weight polymers, vegetable oils and their derivatives and similar plasticizing liquids.

Plasticizing oils preferred for the present invention include naphthenic and paraffinic oils commercially available from a number of suppliers including Shell Chemical Company, Witco, etc. The amount of plasticizing oil is about 10 to 40 percent by weight and preferably about 15 to 35 percent by weight, including any oil that may be incorporated in the block polymer by the supplier.

### Oil Gelling or Complexing Agents

Plasticizing oils used in adhesive compositions can be prevented from migration by the use of gelling or complexing agents which tend to restrain the migration of oil through formations of gels or complexes. The oil can be restrained by a variety of gelling agents including waxes, polyethylene waxes, oxidized waxes, oxidized polyethylene waxes, polyvalent metal soaps, etc.

The following table sets forth useful, preferred, and most preferred formulas.

**Table 1**

| Ingredient | Useful | Preferred | Most Preferred |
|---|---|---|---|
| Radial block copolymer* | 6-25 | 8-22 | 10-19 |
| A-B-A block copolymer* | 0-14 | 0-12 | 0-10 |
| Tackifier | 30-70 | 40-65 | 50-60 |
| Plasticizing oil | 10-40 | 15-35 | 20-30 |
| Synthetic polyethylene wax (or other oil complexing agent) | 0-10 | 0.1-9 | 0.25-5 |

| | | | |
|---|---|---|---|
| *Total polymer content (including both radial block and linear block polymer is equal to or less than about 19 percent by weight of the adhesive. | | | |

### Composite Construction

Broadly, disposable composite articles such as diapers, feminine protection articles, incontinent pads, bed pads and others are typically made by joining to a substrate, typically a plastic or polyolefin film substrate, an absorbent layer frequently covered by a tissue and a woven or nonwoven overlayer.

Plastic substrates useful in the articles of the invention comprise films made from polyethylene, polypropylene, polyester such as polyethylene terephthalate, polyvinyl chloride, polyvinylidine chloride, polyvinyl acetate, and other materials capable of film formation. The tissue layer is a well known, typically loosely formed cellulosic sheet of high porosity or permeability.

The woven or nonwoven layers can consist of a fluid permeable flexible material that can be made of either hydrophilic or hydrophobic fiber components. The woven and nonwoven webs comprising the fabric can comprise natural or synthetic fibers or mixtures thereof. Woven and nonwoven materials are well known and their construction methods have been practiced for many years. Woven fabrics are typically manufactured in weaving machines forming an interlocking mesh of fibers forming the layer. Nonwoven fabrics can be made through a dry laid or wet laid method in carding processes, air laid processes, or spun bond processes to produce a web that is mechanically, chemically or thermally formed. The fabric layers for use in the composite articles of the invention typically have a basis weight in the range of about 8,4 to 20,9 (10 to 25), preferably 11,7 to 15,1 grams to square meter (14 to 18 grams per square yard), a minimum dry tensile of at least 800 grams per centimeter squared in the machine direction, and at least 200 grams per centimeter squared in the cross machine direction.

Synthetic materials commonly used in forming the woven or nonwoven fabric layers include rayon, polyester, polypropylene, polyethylene, nylon and others.

Absorbent layers bonded into the disposable articles of the invention by the adhesive of the invention comprise typically cellulosic pulp or fluff, super-absorbent batts or combinations of fluff and super-absorbent materials. Such fluff layers are often formed and wrapped in tissue to provide mechanical integrity to the fluff which has little inherent mechanical strength. Fluff is typically manufactured through the formation of finely divided cellulosic fibers, however other materials can be used to form highly absorbent fluff or pulp layers.

### Comparative Examples A and B

Using a sigma blade mixer, adhesives for comparative purposes were selected from Raykovitz et al., U.S. Patent No. 4,7044,110 (column 4, formula 3 and the formula from claim 12). The following table shows the compositions of these adhesives.

**Table 2**

| Comparative A | | |
|---|---|---|
| Ingredient | Name | Percent |
| A-B-A block copolymer | Stereon 845 A | 27.5 |
| Plastizing Oil | 500 Second Oil | 7.5 |
| Tackifying resin | Wingtack 95 | 10.0 |
| Tackifying resin | Zonatec 105 Lite | 55.0 |
| Antioxidant | Irganox 1010 | 0.25 |
| Antioxidant | Irgaphos 168 | 0.25 |

| Comparative B | | |
|---|---|---|
| Ingredient | Name | Percent |
| A-B-A block copolymer | Stereon 840 A | 30.0 |
| Plastizing Oil | 500 Second Oil | 20.0 |
| Tackifying resin | Zonatac 105 Lite | 49.5 |
| Antioxidant | Irganox 100 | 0.5 |

### Comparative Example C

Using a sigma blade mixer, an adhesive for comparative purposes was prepared from Collins et al., U.S. Patent No. 4,136,099. The adhesive of Collins et al. is a commercially available positioning adhesive which is the standard of the industry. The following table shows the composition of this adhesive.

**Table 3**

| Ingredient | Name | Percent |
|---|---|---|
| A-B-A block copolymer | Kraton G-1650 | 15.3 |
| Plasticizing oil | 1200 Second Oil | 28.0 |
| Tackifying resin | Wingtack 95 | 53.5 |
| Anti-oxidant | Irganox 1010 | 0.1 |
| Anti-oxidant | Irganox 1076 | 0.1 |
| Pigment | PMS-04110-PHM | 3.0 |

### Example I

A preferred adhesive of the invention was made using a sigma blade mixer and standard blending techniques. The blending techniques outlined in U.S. Patent No. 5,024,667 are preferred for the present invention and are incorporated herein by reference.

**Table 4**

| Example 1 | | |
|---|---|---|
| Ingredient | Name | Percent |
| Radial Block Copolymer | Europene Sol T 161C | 15.0 |
| Plasticizing oil | 500 Second Oil | 24.5 |
| Tackifying resin | Eastotac H-100R Hydrocarbon | 55.0 |
| Antioxidant | Irganox 1010 | 0.5 |
| Polyethylene | Polyethylene AC-8 | 3.0 |
| Pigment | PMS-04110-PHM Pigment | 2.0 |

| Example 2 | | |
|---|---|---|
| Ingredient | Name | Percent |
| Radial Block Copolymer | Sol T 161C | 15.5 |
| Plasticizing oil | 500 Second Oil | 25.0 |
| Tackifying resin | Eastotac H-100R Hydrocarbon | 56.0 |
| Antioxidant | Irganox 1010 | 0.5 |
| Polyethylene | Polyethylene AC-8 | 3.0 |

### Test Procedures

### T-Peel to Cotton

The hot melt adhesives are prepared as films on Mylar™ brand film or polyethylene film using the Acumeter™ or Meltex™ Coater at an appropriate application temperature. The coat weight is 50 gm/m² ± 3gm/m². The coating method employed should be consistent in that significant deviations of test results may occur depending on the method of coating.

### Mylar Coated Samples

The test cotton should be relatively smooth. The test cotton used was style 437 bleached cotton T-shirt fabric. Examination of the test cotton used showed a gridwork of stitching. If a small sample was stretched, one direction of the sample was easy to elongate, and the other direction was comparatively difficult to stretch. The cotton samples were cut lengthwise in the difficult to stretch direction. All specimens were cut as straight as possible along the gridwork of fibers. If the cotton strips were cut askew, the result was an inconsistent elongation of the test strip.

Strips of the test cotton were cut 3,8 cm x 12,7 cm (1 1/2 inches 5 inches). The adhesive film was cut into strips 2,54 cm x 10,2 cm (1 inch x 4 inches), and gently placed on the surface of the test cotton such that the cotton curled away from the bond in the lengthwise direction. Care was taken not to press the adhesive down onto the cotton fabric. The composite was placed on the mechanical roll-down device, and the roller was allowed two passes over the sample, one forward and one back. A timer was activated and the sample was placed into the jaws of a slip peel tester. The adhesive film was placed into the jaw which was attached to the platten, and the cotton was attached to the jaw which was connected to a load cell. No more than one minute passed after the sample was removed from the roll down device and placed into the test jaws. The sample was peeled at 30,5 cm (12 inches) per minute, averaged over 10 seconds. The procedure was repeated five times, recording the average T-peel value. Delayed peels are tested according to the same procedure as initial peels except that after the timer is started, the peels are not tested until one hour and 45 minutes has elapsed. The stressed peel test is conducted using the following procedure: The nylon used is called brushed nylon and is available from Test Fabrics, Inc., P.O. Blackford Avenue, Middlesex, New Jersey 08846. The dimensions of the fabric strips and adhesive/Mylar are the same as for the initial peel testing. The adhesive/Mylar is gently placed on to the surface of the brushed nylon fabric and this composite is placed between two plexiglass plates. This is then placed into a 40,6°C (105° F) oven and a 200 gm. weight placed on top. A timer is then activated and the samples are conditioned for 45 minutes. AFter 45 minutes, the weights are removed and the samples are conditioned between the plates at 23°C and 50% relative humidity for 15 minutes and then peeled in the same manner as initial peel testing.

### Viscosity Tests

The viscosity was tested using a Brookfield Viscometer Model RVTD using a Spindle #27. The rpms were varied depending on the temperature and product.

The variance was as follows:

| | | |
|---|---|---|
| Examples 1 & 2 | @ 135°C (275°F) | 10 rpm |
| | @ 148,9°C (300°F) | 20 rpm |
| | @ 162,8°C (325°F) | 50 rpm |
| | @ 176,7°C (350°F) | 50 rpm |
| Comparative A | @ 135,0°C (275°F) | 5 rpm |
| | @ 148,9°C (300°F) | 10 rpm |
| | @ 162,8°C (325°F) | 20 rpm |
| | @ 176,7°C (350°F) | 50 rpm |
| Comparative B | @ 135,0°C (275°F) | 10 rpm |
| | @ 148,9°C (300°F) | 10 rpm |
| | @ 162,8°C (325°F) | 20 rpm |
| | @ 176,7°C (350°F) | 50 rpm |
| Comparative C | @ 135,0°C (275°F) | 2.5 rpm |
| | @ 148,9°C (300°F) | 10 rpm |
| | @ 162,8°C (325°F) | 50 rpm |
| | @ 176,7°C (350°F) | 100 rpm |

As discussed above, the flatter the viscosity profile over a given range of temperatures, the easier it is for processing and manufacturing. It is generally preferred to have a viscosity change of less than about 7,000 mPas (cps) over a temperature range of 148,9°C to 176,7°C (300°F to 350°F). It is more preferred to have a viscosity change of less than above 6,000 mPas (cps) over a temperature range of 148,9°C to 176,7°C (300°F to 350°F). It is most preferred to have a viscosity change of less than 5,000 mPa·s (cps) over a temperature range of 148,9°C to 176,7°C (300°F to 350°F).

### Peel and Viscosity Results

Adhesive samples were prepared in a method as described above, producing samples having a thickness of about 51 µm (2 mils). Initial peels to cotton and nylon were recorded using the T-peel method described above.

The adhesives of Comparative Examples A, B, C, and Examples 1 and 2 were tested for viscosity, cotton and nylon peels, stressed nylon peel and delayed cotton peel. The results of the testing is shown in Table 5.

**TABLE 5**

| | Example 1 | Example 2 | Comparative A | Comparative B | Comparative C |
|---|---|---|---|---|---|
| Viscosity (mPa·s) (cps) | | | | | |
| @ 135,0°C (275°F) | 13,875 | 15,000 | 29,800 | 21,650 | 75,000 |
| @ 148,9°C (300°F) | 6,925 | 7,150 | 13,900 | 12,375 | 17,000 |
| @ 162,8°C (325°F) | 4,025 | 4,215 | 7,650 | 7,315 | 4,500 |
| @ 176,7°C (350°F) | 2,615 | 2,710 | 4,700 | 4,840 | 1,850 |
| Initial Peel to Cotton | 451±22 | 385±12 | 164±25 | 526±52 | 451±40 |
| Initial Peel to Nylon | 518±30 | 552±56 | 108±11 | 806±111 | 526±47 |
| Stressed Peel to Nylon | 264±57 | 267±60 | 1641±240 | 1531±143 | 333±33 |
| Delayed Peel to Cotton | 184±75 | 180±29 | 185±15 | 522±39 | 277±17 |

The peel values of Examples 1 and 2 are similar to those of Comparative Example C. Comparative C is a standard for the industry for positioning adhesives. Comparative A and B were found to have stressed peels to nylon that were much too high. Comparative A exhibited initial peels to cotton and nylon that were too low. Comparative B exhibited initial peels to nylon that were too high and the product showed transfer in the stressed peel to nylon test. Transfer is indicated for Comparative B because there was a noticeable level of tack left on the nylon after peeling. Overall, Comparative Examples A and B did not exhibit the correct performance, required by adhesive users and suppliers for a usable positioning adhesive. Examples 1 and 2 are superior over Comparative C because of the viscosity profile. The viscosity curve of Comparative Example C is very steep making it necessary to keep a tightly controlled, and high (176,7°C (350°F)), application temperature. Adhesive users prefer products that can be applied at lower application temperatures. The adhesives of the present invention would enable the user to directly apply the adhesive to lower basis weight polyethylene film instead of transfer coating. Transfer coating involves applying the adhesive to a silicone coated release liner first and subsequently combining the coated release liner to the backsheet material. The adhesives have a greater affinity for the backsheet material and ultimately, when the release liner is removed, the adhesive-remains on the backsheet. Direct application is desirable because of the difficulties involved with coating siliconized substrates at high speeds. Degradation of the adhesive is also retarded at lower application temperatures and the potlife of the adhesive is therefore increased. Degradation of the adhesive causes color and viscosity changes and ultimately decreases the performance of the adhesive. Examples 1 and 2 can be applied at lower temperatures due to the flatter viscosity curves.

Further samples were made and were direct coated onto Mylar™ using a Meltex™ coater. The coat weight for all products was approximately 2 mils. The peels were tested according to the methods described above. The following results shows further peel values for Comparative C and Examples 1 and 2. Aging was not completed for Comparatives A and B because the initial peels indicated that these products would not be suitable for positioning of sanitary napkins or incontinent devices.

The following results show aging tests. The aging was conducted at 48,9°C (120° F). and 50% relative humidity.

**Table 7**

| RESULTS: | Comparative C | Example 1 | Example 2 |
|---|---|---|---|
| Initial | | | |
| Initial Peel to Cotton | 447±40 | 403±20 | 343±20 |
| Initial Peel to Nylon | 449±51 | 545±52 | 464±46 |
| Stressed Peel to Nylon | 314±19 | 309±62 | 302±89 |
| Delayed Peel to Cotton | 258±50 | 175±29 | 149±32 |

| 1 Week | | | |
|---|---|---|---|
| Initial Peel to Cotton | 332±7 | 311±16 | 313±48 |
| Initial Peel to Nylon | 408±45 | 393±47 | 374±41 |
| Stressed Peel to Nylon | 310±88 | 259±43 | 235±7 |
| Delayed Peel to Cotton | 178±28 | 87±40 | 131±13 |

| 2 Weeks | | | |
|---|---|---|---|
| Initial Peel to Cotton | 303±23 | 303±28 | 303±36 |
| Initial Peel to Nylon | 367±58 | 366±44 | 424±38 |
| Stressed Peel to Nylon | 317±39 | 256±28 | 220±27 |
| Delayed Peel to Cotton | 199±15 | 104±16 | 133±26 |

| % Change | | | |
|---|---|---|---|
| Initial Peel to Cotton | -32% | -25% | -12% |
| Initial Peel to Nylon | -26% | -33% | -9% |
| Stressed Peel to Nylon | + 1% | -17% | -27% |
| Delayed Peel to Cotton | -23% | -41% | -11% |

The aging results indicate that the examples of the instant invention age as well as the standard of the industry and as discussed above, have a superior viscosity profile.

The above discussion provides a basis for understanding the spirit and scope of the invention. However, many embodiments of the invention can be made obtaining the benefits of the invention residing in the claims hereinafter appended.

## Claims

1. A hot melt adhesive composition, said adhesive comprising:
about 15 to 25 percent by weight of a radial block copolymer having a molecular weight of at least about 160,000 as measured by GEl Permeation Chromatography relative to polystyrene standards, and having the formula:
(A-B)ₙ-Y
wherein Y is a multivalent coupling agent, A comprises a polyvinyl substituted aromatic block, B comprises a polybutadiene rubbery midblock and n is an interger of 3 to 10;
a compatible tackifying resin and a plasticizing oil characterized in that the composition comprises:
a) about 30 to 70 percent by weight of said compatible tackifying resin selected from the group consisting of a C₅ resin, a styrenated terpene resin, a fully hydrogenated resin, a partially hydrogenated resin, a rosin derivative and mixtures thereof; and
b) about 10 to 40 percent by weight of said plasticizing oil; said adhesive composition providing adhesion strong enough to first position an article to an undergarment, said adhesive allowing said article to be transferred to a second position with substantially no adhesive remaining in said first position.

2. The disposable article of claim 1, wherein the adhesive has a viscosity difference of less than about 7,000 mPa·s (cps) through a temperature range of about 148,9°C to 176,7°C (300°F to 350°F).

3. The adhesive of claim 1 wherein A comprises a polystyrene block and B comprises a hydrogenated or non-hydrogenated polymeric block comprising butadiene, isoprene or mixtures thereof.

4. The adhesive of claim 1 wherein B comprises a polybutadiene block.

5. The adhesive of claim 1 wherein B comprises a polyisoprene block.

6. The adhesive of claim 1 wherein the plasticizing oil comprises a naphthenic, a paraffinic oil or mixtures thereof.

7. The adhesive of claim 1 wherein the adhesive additionally comprises 0.1 to 12 percent by weight of a linear A-B-A block copolymer wherein A comprises a polystyrene block and B comprises a polymeric rubbery midblock comprising butadiene, isoprene, or mixtures thereof and the total copolymer content does not exceed 19 percent by weight.

8. A disposable article comprising the adhesive of claim 1.

## Patentansprüche

1. Eine Heißschmelzkleber-Zusammensetzung, wobei der Kleber umfaßt:
ungefähr 15 bis 25 Gew.-% eines radialen Block-Copolymers mit einem Molekulargewicht von wenigstens ungefähr 160.000, relativ zu Polystyrol-Standards gemessen mittels Gel-Permeations-Chromatographie, mit der Formel:
(A-B)ₙ-Y,
wobei Y ein mehrwertiger Kopplungspartner ist, A einen Polyvinylsubstituierten aromatischen Block umfaßt, B einen gummiartigen Polybutadien-Mittelblock umfaßt und n eine ganze Zahl von 3 bis 10 ist;
ein kompatibles klebrigmachendes Harz und ein plastifizierendes Öl,
**dadurch gekennzeichnet, daß** die Zusammensetzung umfaßt:
a) ungefähr 30 bis 70 Gew.-% des kompatiblen klebrigmachenden Harzes ausgewählt aus einer Gruppe bestehend aus C₅-Harz, styreniertem Terpenharz, vollständig hydriertem Harz, teilweise hydriertem Harz, Colophonium-Derivat und Gemischen hiervon; und
b) ungefähr 10 bis 40 Gew.-% des plastifizierenden Öles; wobei die Klebstoffzusammensetzung mit einer Klebkraft versehen ist, die stark genug ist zu einer ersten Plazierung eines Gegenstandes auf einem Futterstoff, wobei es der Klebstoff es ermöglicht, daß der Gegenstand zu einer zweiten Position transferiert wird, wobei im wesentlichen kein Klebstoff an der ersten Position zurückbleibt.

2. Der Einweg-Artikel nach Anspruch 1, worin der Klebstoff eine Viskositätsdifferenz von weniger als ungefähr 7.000 mPa·s (cps) über einen Temperaturbereich von ungefähr 148,9°C bis 176,7°C (300°F bis 350°F) hat.

3. Der Klebstoff nach Anspruch 1, worin A einen Polystyrol-Block umfaßt und B einen hydrierten oder nicht-hydrierten Polymerblock, umfassend Butadien, Isopren oder Gemische hiervon, umfaßt.

4. Der Klebstoff nach Anspruch 1, worin B einen Polybutadien-Block umfaßt.

5. Der Klebstoff nach Anspruch 1, worin B einen Polyisopren-Block umfaßt.

6. Der Klebstoff nach Anspruch 1, worin das plastifizierende Öl ein naphthenisches Öl, ein paraffinisches Öl oder Gemische hiervon umfaßt.

7. Der Klebstoff nach Anspruch 1, worin der Klebstoff zusätzlich 0,1 bis 12 Gew.-% eines linearen A-B-A-Block-Copolymers umfaßt, worin A einen Polystyrol-Block umfaßt und B einen gummiartigen Polymer-Mittelblock, umfassend Butadien, Isopren oder Gemische hiervon, umfaßt und der Gesamtcopolymer-Gehalt 19 Gew.-% nicht übersteigt.

8. Ein Wegwerf-Artikel umfassend den Klebstoff nach Anspruch 1.

## Revendications

1. Composition adhésive thermofusible, ledit adhésif comprenant :
environ 15 à 25 pour cent en poids d'un copolymère séquencé radial ayant une masse moléculaire d'au moins à peu près 160.000, telle que mesurée par chromatographie par pénétration GE1, relative aux normes sur le polystyrène, et ayant pour formule :
(A-B)ₙ-Y
dans laquelle Y est un agent multivalent de couplage, A comprend un bloc aromatique de substitution de polyvinyle, B comprend un bloc intermédiaire caoutchouteux de polybutadiène, et n est un nombre entier compris entre 3 et 10 ;
une résine compatible d'adhésivité et une huile plastifiante, caractérisée en ce que la composition comprend :
a) environ 30 à 70 pour cent en poids de ladite résine compatible d'adhésivité, sélectionnée à partir du groupe comprenant une résine C₅, une résine de terpène au styrène, une résine à hydrogénation complète, une résine à hydrogénation partielle, un dérivé de colophane, et des mélanges de ceux-ci ; et
b) environ 10 à 40 pour cent en poids de ladite huile plastifiante ; ladite composition adhésive assurant une adhérence suffisamment forte pour placer, dans une première position, un article sur un sous-vêtement, ledit adhésif permettant de déplacer ledit article vers une seconde position sans pratiquement laisser d'adhésif dans ladite première position.

2. Article jetable selon la revendication 1, dans lequel l'adhésif a une différence de viscosité inférieure à environ 7.000 mPa.s (cps) sur une plage de températures d'environ 148,9 °C à 176,7 °C (300 °F à 350 °F).

3. Adhésif selon la revendication 1, dans lequel A comprend un bloc polystyrène et B comprend un bloc polymère hydrogéné ou non hydrogéné, comprenant du butadiène, de l'isoprène ou des mélanges de ceux-ci.

4. Adhésif selon la revendication 1, dans lequel a comprend un bloc polybutadiène.

5. Adhésif selon la revendication 1, dans lequel B comprend un bloc polyisoprène.

6. Adhésif selon la revendication 1, dans lequel l'huile plastifiante comprend une huile naphténique, une huile paraffinique, ou des mélanges de celles-ci.

7. Adhésif selon la revendication 1, dans lequel l'adhésif comprend en outre de 0,1 à 12 pour cent en poids d'un bloc copolymère linéaire A-B-A, dans lequel A comprend un bloc polystyrène et B comprend un bloc intermédiaire caoutchouteux polymérique, comprenant le butadiène, l'isoprène, ou des mélanges de ceux-ci, la teneur totale en copolymères n'excédant pas 19 pour cent en poids.

8. Article jetable comprenant l'adhésif selon la revendication 1.
